# EUROPEAN PATENT SPECIFICATION

(11) **EP 2 175 914 B1**
(45) Date of publication and mention of the grant of the patent: **09.10.2024**
(21) Application number: 08776079.9
(22) Date of filing: 28.07.2008
(51) Int. Cl.: A61M 5/20, A61M 5/32

(54) **INJECTION DEVICE WITH LOCKING MECHANISM FOR SYRINGE CARRIER**
INJEKTIONSVORRICHTUNG MIT ARRETIERMECHANISMUS FÜR EINEN SPRITZENTRÄGER
DISPOSITIF D'INJECTION AVEC MÉCANISME DE VERROUILLAGE POUR UN SUPPORT DE SERINGUE

(30) Priority: 08.08.2007 GB 0715456
(43) Date of publication of application: 21.04.2010
(73) Proprietor: Cilag GmbH International, 6300 Zug (CH)
(72) Inventor: JENNINGS, Douglas Ivan, Royston, Hertfordshire SG8 7XU (GB)
(74) Representative: Carpmaels & Ransford LLP
(86) International application number: PCT/GB2008/002573
(87) International publication number: WO 2009/019436

(56) References cited:
- WO-A1-2007/036676
- WO-A2-2007/066152
- WO-A2-99/10030
- US-A1- 2005 203 466

## Description

### Field of the Invention

The present invention relates to an injection device of the type which has a syringe and which extends the syringe, discharges its contents and then retracts it automatically.

### Background of the Invention

Injection devices are shown in WO 95/35126 and EP-A-O 516 473. These devices employ a drive spring and some form of release mechanism that releases the syringe from the influence of the drive spring once its contents are supposed to have been discharged, to allow it to be retracted by a return spring.

WO2007/066152A2 describes an injection device comprising means for adjusting the amount of substance to be expelled from a container. WO2007/036676A1 describes an injection device having a sleeve which projects from an exit aperture and can be depressed to release a locking mechanism. US2005203466 (A1) describes an autoinjector with active agent container latching. WO9910030 (A2) describes an autoinjector with a re-usable firing mechanism.

Generally, the return spring is relatively weak, since its restoring force must be overcome by the drive spring, even while the drive spring is doing work on the various components of the injection device and the syringe during an injection cycle. This may give rise to a problem when the injection device is used with sealed hypodermic syringes, which typically have a hermetically sealed cover, needle shield or "boot" that covers the hypodermic needle and maintains the sterility of the syringe contents. Naturally, it is necessary to maintain the sterility of the syringe contents up to the point of administration, which devices that are designed to be disposable, as many will be, means that the boot must be removed with the syringe inside the injection device.

Typically, the action required to remove the boot from the syringe is simply to pull the boot away from the syringe, which requires a force in excess of 20N. This is significantly greater than the restoring force of the return spring, so the syringe will be pulled out of the injection device as the boot is removed and, when the boot comes away, it will snap back into place. This is not the best way to handle the syringe. The shock could damage it, the needle could be damaged and there may be problems re-engaging the syringe with those components of the injection device designed to act upon it. Even in cases where there is no return spring, for example where the syringe is held in place by friction with components of the injection device, the problem will still arise of relocating the syringe onto those components of the injection device designed to act upon it.

Moreover, there is a problem with having the syringe generally moveable in a direction out of the injection device. Accidental activation of the drive spring by mechanical failure of the drive spring's release mechanism (e.g. a trigger) can occur, for example by dropping the device on a hard surface. This accidental activation could cause the syringe to be extended unintentionally out of the device and its contents to be ejected. This could expose the needle of the syringe and increase the risk of inadvertent ski puncturing and/or infection.

### Summary of the Invention

The injection device of the present invention is designed to deal with the aforementioned problems. The invention is defined in claim 1. Embodiments of the invention are defined in the dependent claims.

Thus, the syringe carrier and syringe are locked in place within the injection device until such time that the device is actuated by activation of the release mechanism. This prevents damage to the syringe and its contents. Moreover, this assists in preventing accidental activation of the injection device, for example by dropping the injection device on a hard surface.

According to one embodiment of the invention, the aperture may comprise:
a first portion which is dimensioned to engage with the syringe carrier when the release mechanism is in its engaged position; and
a second portion which is dimensioned not to engage with the syringe carrier when the release mechanism is in its disengaged position.

This arrangement provides a secure and effective locking mechanism for preventing movement of the syringe carrier via the release mechanism, in addition to the release mechanism's function of retaining the drive in an unactuated position until such time that the release mechanism is activated.

### Brief Description of the Drawings

The invention will now be described by way of example with reference to the accompanying drawings, in which:
Fig. 1a is a right-side view of the injection device according to the present invention;
Fig. 1b is a perspective view of the injection device of Fig. 1 with its cap removed;
Fig. 1c is a perspective view of the cap of the injection device of Fig. 1;
Fig. 2a is an exploded right-side view of the injection device of Fig. 1;
Fig. 2b is a right-side view of the assembled components of the injection device of Fig. 1;
Fig. 2c is a perspective view of a multi-component drive used in the injection device of Fig. 1
Fig. 3a is a perspective view of a proximal portion of a trigger button used in the injection device of Fig. 1; and
Fig. 3b is a cross-sectional view of the injection device of Fig. 1.

### Detailed Description of the Drawings

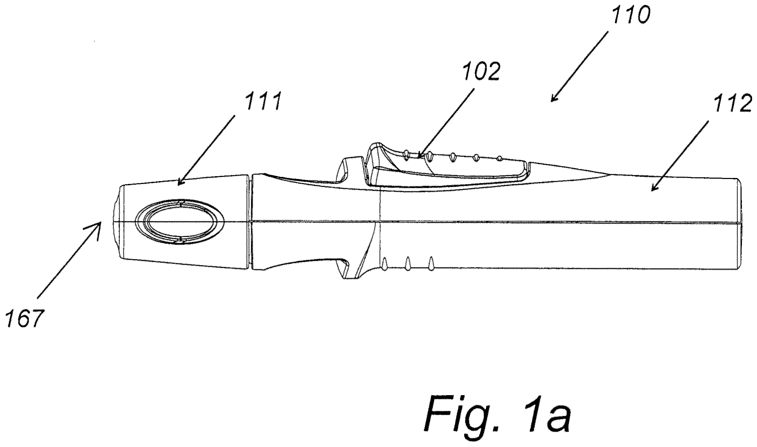

Fig. 1a is a right-side view of an injection device 110 according to the present invention. The injection device 110 has a housing 112, a cap 111 which is removable from a proximal end 167 the housing 112 and a trigger button 102. Other parts of the device will be described in greater detail below.

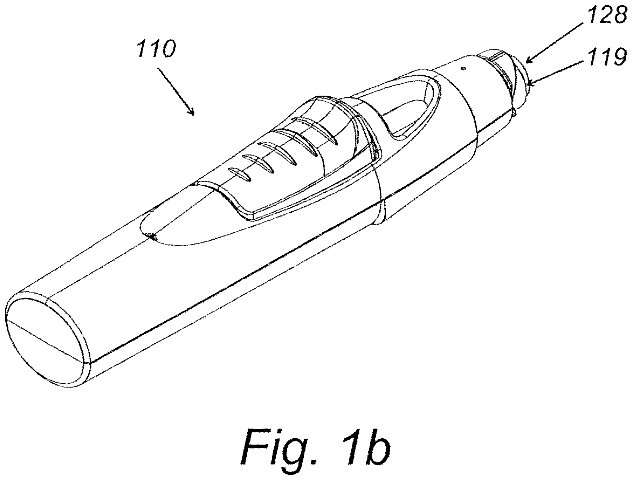

Fig. 1b is a perspective view of the injection device 110 according to the present invention with the cap (not shown) removed from its end. The end of the housing 112 has an exit aperture 128, from which the end of a sleeve 119 can be seen to emerge.

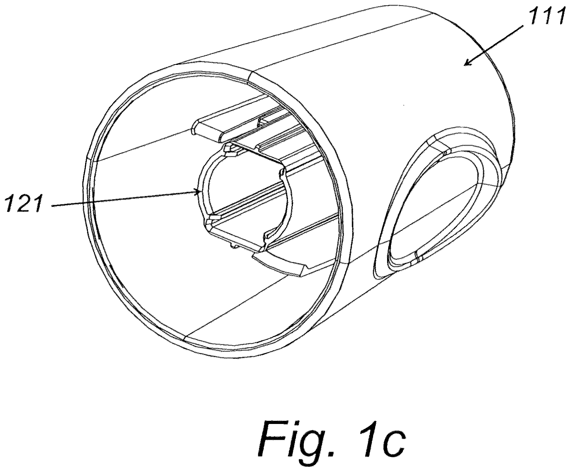

Fig. 1c is a perspective view of the cap 111 of the injection device 110 according to the present invention. The cap 111 has a central boss 121 that fits within the sleeve 119 when the cap 111 is installed on the housing 112.

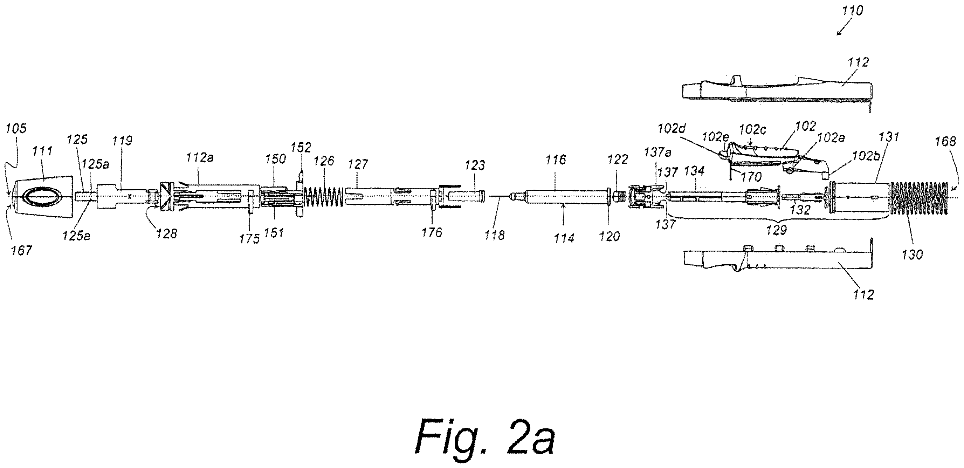

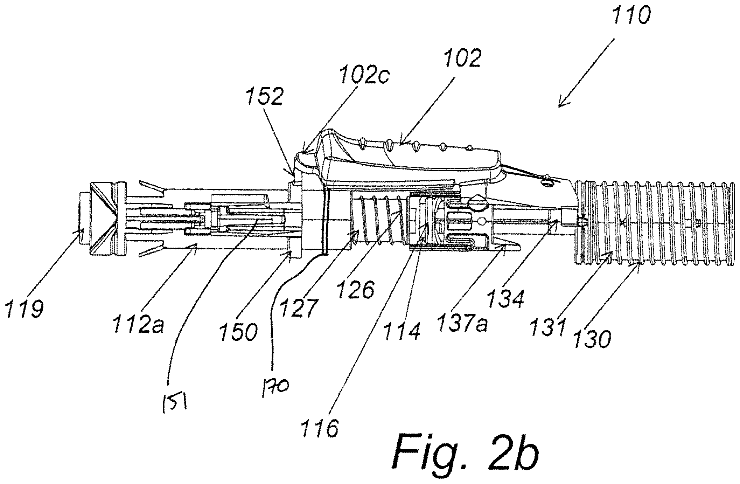

Fig. 2a is an exploded right-side view of the components of the injection device 110 according to the present invention and Fig. 2b is a right-side view of the assembled components of the injection device 110 according to the present invention without the housing 112 or cap 111.

As illustrated, the injection device 110 comprises a hypodermic syringe 114 of conventional type, including a syringe body 116 terminating at one end in a discharge nozzle, specifically a hypodermic needle 118, and at the other in a flange 120. The conventional plunger that would normally be used to discharge the contents of the syringe 114 manually has been removed and replaced with a drive element (referred to below as the second drive element 134) that contacts a bung 122 in the syringe 114. The bung 122 constrains a drug (not shown) to be administered within the syringe body 116. Whilst the syringe illustrated is of hypodermic type, this need not necessarily be so. Transcutaneous or ballistic dermal and subcutaneous syringes may also be used with the injection device of the present invention.

As illustrated, the injection device 110 includes a return spring 126 that biases the syringe 114 from an extended position in which the needle 118 extends from the aperture 128 in a case nose 112a of the housing 112 to a retracted position in which the needle 118 is contained within the housing 112. The return spring 126 acts on the syringe 114 via a syringe carrier 127. The syringe 114 is moveable along a longitudinal axis 105 of the injection device 110 which extends centrally along the length of the injection device 110 from the exit aperture 128 at its proximal end 167 to a distal end 168.

Contained within the housing at its distal end 168 is an actuator, which here takes the form of a compression drive spring 130. Drive from the drive spring 130 is transmitted via a multi-component drive 129 to the syringe 114 to advance it from its retracted position to its extended position and discharge its contents through the needle 118. The drive 129 accomplishes this task by acting directly on the drug and the syringe 114. Hydrostatic forces acting through the drug and, to a lesser extent, static friction between the bung 122 and the syringe body 116 initially ensure that they advance together, until the return spring 126 bottoms out on the syringe carrier 127 or meets some other obstruction (not shown) that retards its motion.

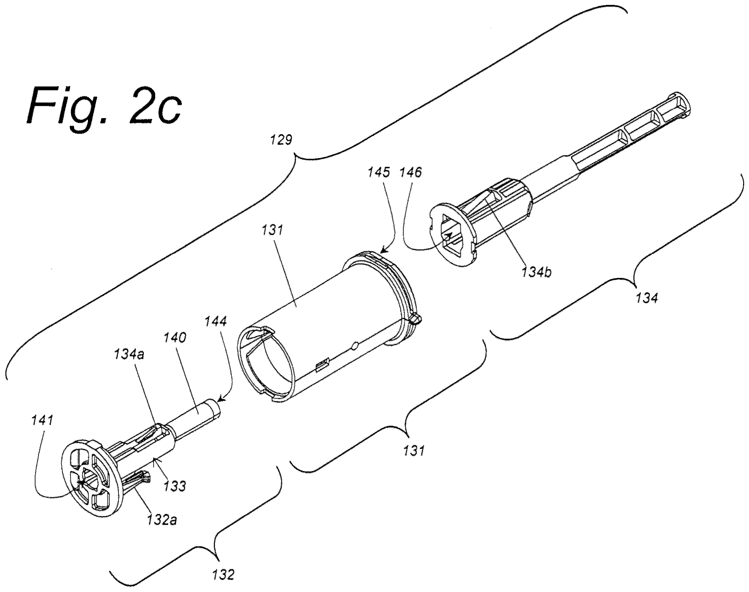

Fig. 2c is an exploded perspective view of the multi-component drive 129. The multi-component drive 129 between the drive spring 130 and the syringe 114 consists of three principal components. A drive sleeve 131 takes drive from the drive spring 130 and transmits it to a delay piston 133 on a first drive element 132. This in turn transmits drive to the second drive element 134.

As will be seen from Fig. 2c, the first drive element 132 includes a hollow stem 140, the inner cavity of which forms a collection chamber 141 in communication with a vent 144 that extends from the collection chamber 141 through the end of the stem 140. The second drive element 134 includes a blind bore 146 that is open at one end to receive the stem 140 and closed at the other. As will be appreciated, the bore 146 and the stem 140 define a fluid reservoir within which a damping fluid is contained.

The trigger button 102 is provided on the side of the housing 112 which, when in an engaged position with a proximal end 145 of the drive sleeve 131, serves to retain the drive spring 130 in a compressed state by contact between locking surface 102b and the drive sleeve 131 when the button 102 is in an unactuated position. The trigger button 102 can pivot on the housing 112 via pivot 102a. When downwards pressure is applied to the trigger button 102 at an activation surface 102c (i.e. pressure directed into the housing 112), the locking surface 102b moves upwards in a direction away from the longitudinal axis 105. In this actuated position of the button 102, the locking surface 102b is decoupled from the drive sleeve 131, thereby allowing the drive sleeve 131 to move relative to the housing 112 towards the exit aperture 128 under the influence of the drive spring 130.

The sliding sleeve 119 is moveable from its extended position (as shown in Fig. 1b) where it protrudes out of the exit aperture 128 into a retracted position in the case nose 112a of the housing 112. The sliding sleeve 119 is connected to a button lock element 150 which has resilient arms 151 which bias the sliding sleeve 119 into its extended position in which its end protrudes from the end of the case nose 112a. Thus, application of pressure to the end of the sliding sleeve 119, for example by pressing the end of the sliding sleeve 119 against tissue, causes it to move into its retracted position into the housing 112; release of the pressure causes the sliding sleeve 119 to move into its extended position under bias from the resilient arms 151 acting against a side wall of the housing 112. The button lock element 150 has a button lock protrusion 152 which contacts with the end of a trigger button protrusion 102d on the trigger button 102 when the sliding sleeve is in its extended position. The trigger button protrusion 102 extends in a direction which is generally parallel to the longitudinal axis 105 of the injection device 110. The button lock protrusion 152 extends in a direction which is generally perpendicular to the longitudinal axis 105 towards the trigger button protrusion 102d. The trigger button protrusion 102d has an aperture 102e which can move over the top of the button lock protrusion 152 when the button lock element 150 has been moved away from the exit aperture 128 (i.e. when the sliding sleeve 119 has been moved into the exit aperture 128 into its retracted position). In this position, the trigger button 102 can be moved into its deactivated position by rotating the trigger button 102 about the pivot 102a in the direction of the pressure applied to the pressure surface 102c. Thus, the button lock element 150 and the sliding sleeve 119 act together to lock the trigger button 102 in its activated position (i.e. the locking surface 102b contacts the end of the drive sleeve 131 preventing it from moving towards the exit aperture 128 under the bias of the compressed drive spring 130).

When the sliding sleeve 119 has been moved into a position in which it is retracted into the housing 112 (i.e. into its unlocked position) and the trigger button 102 has been rotated into its deactivated position, the operation of the device 110 is then as follows.

Initially, the drive spring 130 moves the drive sleeve 131, the drive sleeve 131 moves the first drive element 132 and the first drive element 132 moves the second drive element 134, in each case by acting through flexible latch arms 132a, 134a, 134b. The second drive element 134 moves and, by virtue of static friction and hydrostatic forces acting through the drug (not shown), moves the syringe body 116 and syringe carrier 127 against the action of the return spring 126. The return spring 126 compresses and the hypodermic needle 118 emerges from the exit aperture 128 of the housing 112. This continues until the return spring 126 bottoms out or the syringe body 116 meets some other obstruction (not shown) that retards its motion. Because the static friction between the second drive element 134 and the syringe body 116 and the hydrostatic forces acting through the drug (not shown) to be administered are not sufficient to resist the full drive force developed by the drive spring 130, at this point the second drive element 134 begins to move within the syringe body 116 and the drug (not shown) begins to be discharged. Dynamic friction between the second drive element 134 and the syringe body 116 and hydrostatic forces acting through the drug (not shown) to be administered are, however, sufficient to retain the return spring 126 in its compressed state, so the hypodermic needle 118 remains extended.

Before the second drive element 134 reaches the end of its travel within the syringe body 116, so before the contents of the syringe have fully discharged, the flexible latch arms 134a, 134b linking the first and second drive elements 132, 134 reach a constriction 137 provided on a latch actuator element 137a which is fixed to the end of the syringe carrier 127. The constriction 137 moves the flexible latch arms 134a, 134b inwards from the position shown in Fig. 2c to a position at which the flexible latch arms 134a, 134b no longer couple the first drive element 132 to the second drive element 134, aided by the bevelled surfaces on the constriction 137. Once this happens, the first drive element 132 acts no longer on the second drive element 134, allowing the first drive element 132 to move relative to the second drive element 134.

Because the damping fluid is contained within a reservoir (not shown) defined between the end of the first drive element 132 and the blind bore 146 in the second drive element 134, the volume of the reservoir will tend to decrease as the first drive element 132 moves relative to the second drive element 134 when the former is acted upon by the drive spring 130. As the reservoir collapses, damping fluid is forced through the vent 144 into the collection chamber 141. Thus, once the flexible latch arms 134a, 134b have been released, the force exerted by the drive spring 130 does work on the damping fluid, causing it to flow though the constriction formed by the vent 144, and also acts hydrostatically through the fluid and through friction between the first and second drive elements 132, 134, thence via the second drive element 134. Losses associated with the flow of the damping fluid do not attenuate the force acting on the body of the syringe to a great extent. Thus, the return spring 126 remains compressed and the hypodermic needle remains extended.

After a time, the second drive element 134 completes its travel within the syringe body 116 and can go no further. At this point, the contents of the syringe 114 are completely discharged and the force exerted by the drive spring 130 acts to retain the second drive element 134 in its terminal position and to continue to cause the damping fluid to flow though the vent 144, allowing the first drive element 132 to continue its movement.

Before the reservoir of fluid is exhausted, the flexible latch arms 132a linking the drive sleeve 131 with the first drive element 132 reach another constriction (not shown) within the housing 112. This constriction moves the flexible latch arms 132a inwards from the position shown to a position at which they no longer couple the drive sleeve 131 to the first drive element 132, aided by bevelled surfaces on the constriction. Once this happens, the drive sleeve 131 acts no longer on the first drive element 132, allowing them to move relative each other. At this point, of course, the syringe 114 is released, because the forces developed by the drive spring 130 are no longer being transmitted to the syringe 114, and the only force acting on the syringe will be the return force from the return spring 126. Thus, the syringe 114 is now returned to its retracted position and the injection cycle is complete.

All this takes place, of course, only once the cap 111 has been removed from the end of the housing 112. The end of the syringe is sealed with a boot 123. The central boss 121 of the cap that fits within the sleeve 119 when the cap 111 is installed on the housing 112 comprises a retainer element 125 which is fixed into the boss 121. The retainer element 125 comprises resilient protrusions 125a which are directed away from the exit aperture 128. These resilient protrusions 125a deform as the cap 111 is inserted onto the housing 112 over a needle shield or rubber boot 123. The protrusions 125a then grip the boot 123 tightly so that the ends of the protrusions are slightly embedded in the boot 123 which might be made from rubber. This means that, as the cap 111 is pulled off the housing 112, the boot 123 is pulled away from the syringe 114 with the cap 111.

Fig. 2a also shows a syringe lock protrusion 170 located on the button 102 at its distal end which is proximal to the end which is located nearest to the aperture 128. The syringe lock protrusion 170 extends in a generally perpendicular direction (with respect to the longitudinal axis 105) into the injection device 110 towards the longitudinal axis 105.

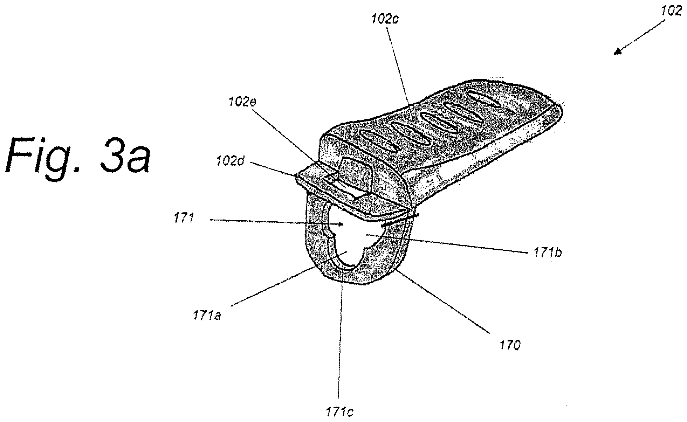

Fig. 3a illustrates a distal end of the button 102 in greater detail. As can be seen, the syringe lock protrusion 170 comprises an aperture 171 which includes a first portion 171a and a second portion 171b. The first and second portions 171a, 171b overlap with each other and have different cross-sectional areas as will be seen from Fig. 3a. The first portion 171a has an edge 171c.

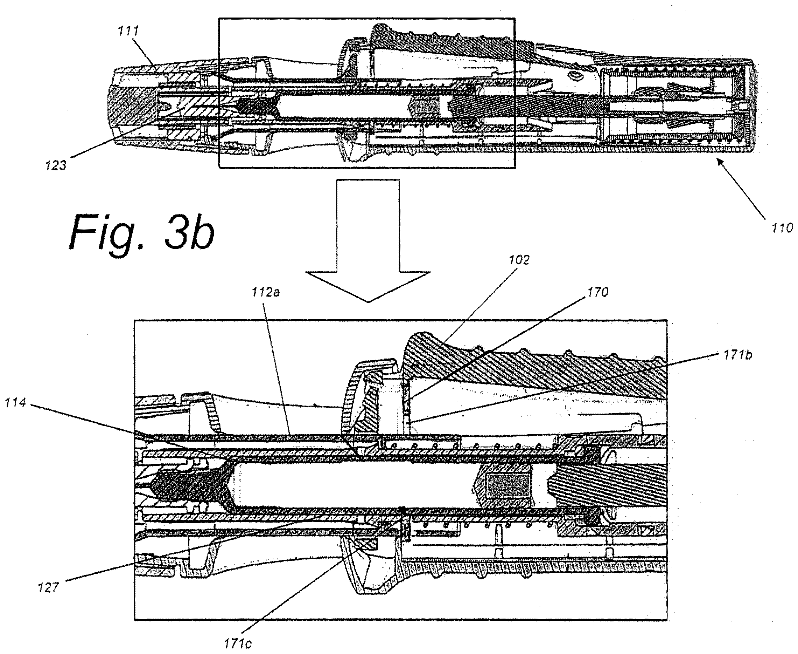

Fig. 3b shows how the button 102 is integrated with the injection device 110 of the present invention.

The case nose 112a comprises a case nose slot 175 located towards the distal end of the housing 112. The case nose slot 175 extends about a substantial proportion of the circumference of the case nose 112a and extends through the case nose 112a. The case nose slot 175 does not extend round the case nose 112a on a section of its circumference which faces towards the button 102. The length of this section about the circumference of the case nose 112a corresponds to the overlap between the first and second portions 171a, 171b of the syringe lock protrusion 170. The width of the case nose slot 175 (in a direction along the longitudinal axis 105) is slightly more than the thickness of the edge 171c of the syringe lock protrusion 170.

The syringe carrier 127 comprises a syringe carrier slot 176 located towards the distal end of the syringe carrier 127. The syringe carrier slot 176 extends about a substantial proportion of the circumference of the syringe carrier 127 and extends through the syringe carrier 127 (although this is not absolutely necessary). The syringe carrier slot 176 does not extend round the syringe carrier 127 on a section of its circumference which faces towards the button 102. The length of this section about the circumference of the syringe carrier 127 corresponds to the overlap between the first and second portions 171a, 171b of the syringe lock protrusion 170. As with the case nose slot 175, the width of the syringe carrier slot 176 (in a direction along the longitudinal axis 105) is slightly more than the thickness of the edge 171c of the syringe lock protrusion 170.

In the unactuated position of the button 102 (as shown in Fig. 3b), the first portion 171a of the syringe lock protrusion 170 surrounds the case nose slot 175 and the syringe carrier slot 176. In addition, the edge 171c of the syringe lock protrusion 170 extends through the case nose slot 175 into the syringe carrier slot 176 so that the syringe carrier 127 is locked to the button 102 and cannot move along the longitudinal axis 105. This prevents the syringe 114 and syringe carrier 127 moving towards the exit aperture 128 when the cap 111 is removed or through accidental activation of the injection device 110, for example by dropping it on a hard surface.

When the button 102 is moved to its actuated position, the edge 171c of the first portion 171a of the syringe lock protrusion 170 moves out of the syringe carrier slot 176 so that the second portion 171b of the syringe carrier protrusion 170 surrounds the syringe carrier 127, but does not engage with the syringe carrier slot 176. In this way, the syringe carrier 127 is no longer locked to the button 102 and can move along the longitudinal axis 105. Thus, the syringe 114 will extend under bias from the drive spring 130 along the longitudinal axis 105 because activation of the button 102 (by applying force to its pressure surface 102c) will have released the drive cylinder 131 from its contacting position against the locking surface 102b, thereby permitting the multi-component drive to move towards the exit aperture 128, along with the syringe 114 and syringe carrier 127.

Thus, the syringe 114 and syringe carrier 127 are prevented from moving longitudinally until the time that the button 102 is actuated. Of course, this also requires that the sliding sleeve 119 has also been moved into its retracted position, thereby releasing the button lock element 150 from its locked position against the button 102.

It will of course be understood that the present invention has been described above purely by way of example and modifications of detail can be made within the scope of the invention, which is defined by the claims.

## Claims

1. An injection device (110) comprising:
a housing (112) adapted to receive a syringe (114) having a discharge nozzle (118), the syringe being moveable in the housing along a longitudinal axis between a retracted position in which the discharge nozzle is contained within the housing and an extended position in which the discharge nozzle of the syringe extends from the housing through an exit aperture (128);
an actuator (130);
a drive (129) adapted to be acted upon by the actuator (130) and in turn act upon the syringe to advance it from its retracted position to its extended position and discharge its contents through the discharge nozzle;
a release mechanism comprising a trigger button (102) adapted, in an engaged position, to prevent the actuator (130) acting on the drive (129) and, in a disengaged position, to permit the actuator (130) to act on the drive (129);
a syringe carrier (127) adapted to support the syringe as it is advanced; and
**characterised by**
a locking mechanism (170) between the syringe carrier and the release mechanism to inhibit movement of the syringe carrier (127) and syringe (114) towards the exit aperture when the trigger button (102) is in its engaged position, wherein the locking mechanism comprises a protrusion (170), located on the trigger button (102) at its distal end which is proximal to the end which is located nearest to the aperture (128), the protrusion (170) extending in a perpendicular direction with respect to the longitudinal axis (105) into the housing towards the longitudinal axis (105),
wherein the protrusion (170) engages the syringe carrier (127) in the engaged position of the trigger button (102) and is disengaged from the syringe carrier (127) in the disengaged position of the trigger button (102).

2. The injection device according to claim 1, wherein the locking mechanism is adapted to prevent movement of the syringe carrier towards the exit aperture when the trigger button is in its engaged position.

3. The injection device of claim 1 or claim 2, wherein the trigger button is located at an external side surface of the housing.

4. The injection device according to claim 3, wherein the external side surface is displaced from the longitudinal axis in a perpendicular direction with respect to the longitudinal axis.

5. The injection device according to any one of the preceding claims, wherein the trigger button comprises an activation surface (102c) adapted for application of pressure by a user of the injection device in a direction into the housing to move the trigger button from its engaged position to its disengaged position.

6. The injection device of claim 1, wherein the protrusion comprises an aperture (171) through which the syringe carrier extends.

7. The injection device of claim 6, wherein the aperture comprises:
a first portion (171a) which is dimensioned to engage with the syringe carrier when the trigger button is in its engaged position; and
a second portion (171b) which is dimensioned not to engage with the syringe carrier when the trigger button is in its disengaged position.

8. The injection device of claim 7, wherein an edge (171c) of the first portion of the aperture engages with the syringe carrier when the trigger button is in its engaged position and is not so engaged when the trigger button is moved to its disengaged position.

9. The injection device of any one of claims 1 to 8, wherein the syringe carrier comprises an opening for engagement by the protrusion when the locking mechanism is in its engaged position.

10. The injection device of claim 9, wherein the opening is a channel extending about a section of the circumference of the outer surface of the syringe carrier.

11. The injection device of claim 9, wherein the opening is a slot about a section of the circumference of the outer surface of the syringe carrier and wherein the slot extends through the outer surface of the syringe carrier.

12. The injection device of any one of claims 1 to 11, wherein a section of the housing comprises an opening through which the protrusion extends, at least when the locking mechanism is in its engaged position.

13. The injection device of any one of the preceding claims, wherein the drive comprises a shaft extending parallel to the longitudinal axis.

14. The injection device of any one of the preceding claims, wherein the actuator comprises biasing means adapted to bias the syringe carrier from a retracted position to an extended position.

15. The injection device of any one of the preceding claims, further comprising a removable cap (111) for locating on the housing and for covering at least a portion of the exit aperture.

16. The injection device of claim 15, wherein the removable cap is connected to a removable shield (123) on the discharge nozzle, such that the shield is removed from the discharge nozzle when the cap is removed from the housing.

17. The injection device according to any one of the preceding claims, wherein the drive includes first and second drive elements (132,134), of which the first is acted upon by the actuator and in turn acts upon the second, and the second acts upon the syringe or the syringe carrier to advance it from its retracted position to its extended position and discharge its contents through the discharge nozzle, the first drive element being capable of movement relative to the second when the first is acted upon by the actuator and the second is restrained by the syringe or the syringe carrier.

18. The injection device according to claim 17, further comprising a coupling that prevents the first drive element from moving relative to the second until they have been advanced to a nominal decoupling position that is less advanced than the said nominal release position.

19. The injection device according to claim 17, wherein the coupling comprises a decoupling mechanism, activated when the drive elements have been advanced to the said nominal decoupling position and adapted to decouple the first drive element from the second, thus allowing the first drive element to move relative to the second.

## Patentansprüche

1. Injektionsvorrichtung (110), umfassend:
ein Gehäuse (112), das zur Aufnahme einer Spritze (114) mit einer Auslassdüse (118) geeignet ist, wobei die Spritze in dem Gehäuse entlang einer Längsachse zwischen einer eingefahrenen Position, in der sich die Auslassdüse im Gehäuse befindet, und einer ausgefahrenen Position, in der die Auslassdüse der Spritze durch eine Austrittsöffnung (128) aus dem Gehäuse herausragt, bewegbar ist;
einen Aktuator (130);
einen Antrieb (129), der so ausgelegt ist, dass er vom Aktuator (130) betätigt wird und seinerseits auf die Spritze einwirkt, um sie aus ihrer eingefahrenen Position in ihre ausgefahrene Position vorzuschieben und ihren Inhalt durch die Auslassdüse abzugeben;
einen Freigabemechanismus, der einen Auslöseknopf (102) umfasst, der in einer eingerückten Position dazu geeignet ist, zu verhindern, dass der Aktuator (130) auf den Antrieb (129) einwirkt, und der in einer ausgerückten Position dazu geeignet ist, zuzulassen, dass der Aktuator (130) auf den Antrieb (129) einwirkt;
einen Spritzenträger (127), der dazu geeignet ist, die Spritze beim Vorschieben zu stützen; und
**gekennzeichnet dadurch, dass**
ein Verriegelungsmechanismus (170) zwischen dem Spritzenträger und dem Freigabemechanismus die Bewegung des Spritzenträgers (127) und der Spritze (114) in Richtung der Austrittsöffnung zu verhindern, wenn sich der Auslöseknopf (102) in seiner eingerückten Position befindet, wobei der Verriegelungsmechanismus einen Vorsprung (170) umfasst, der sich an dem Auslöseknopf (102) an seinem distalen Ende befindet, das proximal zu dem Ende liegt, das sich am nächsten an der Öffnung (128) befindet, wobei sich der Vorsprung (170) in einer senkrechten Richtung in Bezug auf die Längsachse (105) in das Gehäuse in Richtung der Längsachse (105) erstreckt,
wobei der Vorsprung (170) in der eingerückten Position des Auslöseknopfs (102) mit dem Spritzenträger (127) in Eingriff steht und in der ausgerückten Position des Auslöseknopfs (102) von dem Spritzenträger (127) gelöst ist.

2. Injektionsvorrichtung nach Anspruch 1, wobei der Verriegelungsmechanismus dazu ausgelegt ist, eine Bewegung des Spritzenträgers in Richtung der Ausgangsöffnung zu verhindern, wenn sich der Auslöseknopf in seiner eingerückten Position befindet.

3. Injektionsvorrichtung nach Anspruch 1 oder Anspruch 2, wobei sich der Auslöseknopf an einer Außenseitenoberfläche des Gehäuses befindet.

4. Injektionsvorrichtung nach Anspruch 3, wobei die Außenseitenoberfläche in einer senkrechten Richtung bezüglich der Längsachse von der Längsachse versetzt ist.

5. Injektionsvorrichtung nach einem der vorstehenden Ansprüche, wobei der Auslöseknopf eine Aktivierungsoberfläche (102c) umfasst, die so ausgebildet ist, dass ein Benutzer der Injektionsvorrichtung in einer Richtung in das Gehäuse hinein Druck ausüben kann, um den Auslöseknopf aus seiner eingerückten Position in seine ausgerückte Position zu bewegen.

6. Injektionsvorrichtung nach Anspruch 1, wobei der Vorsprung eine Öffnung (171) umfasst, durch die sich der Spritzenträger erstreckt.

7. Injektionsvorrichtung nach Anspruch 6, wobei die Öffnung umfasst:
einen ersten Abschnitt (171a), der so bemessen ist, dass er mit dem Spritzenträger in Eingriff kommt, wenn sich der Auslöseknopf in seiner eingerückten Position befindet; und
einen zweiten Abschnitt (171b), der so bemessen ist, dass er nicht mit dem Spritzenträger in Eingriff tritt, wenn sich der Auslöseknopf in seiner ausgerückten Position befindet.

8. Injektionsvorrichtung nach Anspruch 7, wobei eine Kante (171c) des ersten Abschnitts der Öffnung mit dem Spritzenträger in Eingriff steht, wenn sich der Auslöseknopf in seiner eingerückten Position befindet, und nicht in Eingriff steht, wenn der Auslöseknopf in seine ausgerückte Position bewegt wird.

9. Injektionsvorrichtung nach einem der Ansprüche 1 bis 8, wobei der Spritzenträger eine Öffnung zum Eingreifen durch den Vorsprung umfasst, wenn sich der Verriegelungsmechanismus in seiner eingerückten Position befindet.

10. Injektionsvorrichtung nach Anspruch 9, wobei die Öffnung ein Kanal ist, der sich über einen Abschnitt des Umfangs der Außenoberfläche des Spritzenträgers erstreckt.

11. Injektionsvorrichtung nach Anspruch 9, wobei die Öffnung ein Schlitz um einen Abschnitt des Umfangs der Außenoberfläche des Spritzenträgers ist und wobei der Schlitz durch die Außenoberfläche des Spritzenträgers verläuft.

12. Injektionsvorrichtung nach einem der Ansprüche 1 bis 11, wobei ein Abschnitt des Gehäuses eine Öffnung umfasst, durch die sich der Vorsprung erstreckt, zumindest wenn sich der Verriegelungsmechanismus in seiner eingerückten Position befindet.

13. Injektionsvorrichtung nach einem der vorstehenden Ansprüche, wobei der Antrieb eine parallel zur Längsachse verlaufende Welle umfasst.

14. Injektionsvorrichtung nach einem der vorstehenden Ansprüche, wobei der Aktuator Vorspannmittel umfasst, die dazu geeignet sind, den Spritzenträger aus einer eingefahrenen Position in eine ausgefahrene Position vorzuspannen.

15. Injektionsvorrichtung nach einem der vorstehenden Ansprüche, ferner umfassend eine abnehmbare Kappe (111) zum Positionieren auf dem Gehäuse und zum Abdecken von mindestens einem Abschnitt der Austrittsöffnung.

16. Injektionsvorrichtung nach Anspruch 15, wobei die abnehmbare Kappe mit einem abnehmbaren Schild (123) an der Auslassdüse verbunden ist, so dass der Schutzschild von der Auslassdüse entfernt wird, wenn die Kappe vom Gehäuse entfernt wird.

17. Injektionsvorrichtung nach einem der vorstehenden Ansprüche, wobei der Antrieb ein erstes und ein zweites Antriebselement (132, 134) einschließt, von denen das erste durch den Aktuator beaufschlagt wird und wiederum auf das zweite wirkt, und das zweite auf die Spritze oder den Spritzenträger wirkt, um diese aus ihrer eingefahrenen Position in ihre ausgefahrene Position zu bewegen und ihren Inhalt durch die Auslassdüse auszugeben, wobei das erste Antriebselement sich relativ zum zweiten bewegen kann, wenn das erste durch den Aktuator beaufschlagt wird und das zweite durch die Spritze oder den Spritzenträger zurückgehalten wird.

18. Injektionsvorrichtung nach Anspruch 17, ferner umfassend eine Kupplung, die eine Bewegung des ersten Antriebselements relativ zum zweiten verhindert, bis diese in eine nominale Entkupplungsposition vorgeschoben wurden, die weniger weit vorgeschoben ist als die nominale Freigabeposition.

19. Injektionsvorrichtung nach Anspruch 17, wobei die Kupplung einen Entkupplungsmechanismus umfasst, der aktiviert wird, wenn die Antriebselemente in die nominale Entkupplungsposition vorgeschoben wurden, und der dazu geeignet ist, das erste Antriebselement vom zweiten zu entkuppeln, wodurch eine Bewegung des ersten Antriebselements relativ zum zweiten ermöglicht wird.

## Revendications

1. Dispositif d'injection (110) comprenant :
un boîtier (112) adapté pour recevoir une seringue (114) ayant une buse de décharge (118), la seringue pouvant être déplacée dans le boîtier le long d'un axe longitudinal entre une position rétractée dans laquelle la buse de décharge est contenue dans le boîtier et une position étendue dans laquelle la buse de décharge de la seringue s'étend à partir du boîtier par une ouverture de sortie (128) ;
un actionneur (130) ;
un entraînement (129) adapté pour être actionné par l'actionneur (130) et agir à son tour sur la seringue pour la faire avancer de sa position rétractée à sa position étendue et décharger son contenu par la buse de décharge ;
un mécanisme de libération comprenant un bouton de déclenchement (102) adapté, dans une position en prise, pour empêcher l'actionneur (130) d'agir sur l'entraînement (129) et, dans une position désolidarisée, pour permettre à l'actionneur (130) d'agir sur l'entraînement (129) ;
un porte-seringue (127) adapté pour soutenir la seringue à mesure de son avancement ; et
**caractérisé par**
un mécanisme de verrouillage (170) entre le porte-seringue et le mécanisme de libération pour empêcher le mouvement du porte-seringue (127) et de la seringue (114) vers l'ouverture de sortie lorsque le bouton de déclenchement (102) est dans sa position en prise, dans lequel le mécanisme de verrouillage comprend une saillie (170), située sur le bouton de déclenchement (102) à son extrémité distale qui est proximale par rapport à l'extrémité qui est la plus proche de l'ouverture (128), la saillie (170) s'étendant dans une direction perpendiculaire à l'axe longitudinal (105) dans le boîtier vers l'axe longitudinal (105),
dans lequel la saillie (170) vient en prise avec le porte-seringue (127) dans la position en prise du bouton de déclenchement (102) et est désolidarisée du porte-seringue (127) dans la position désolidarisée du bouton de déclenchement (102).

2. Dispositif d'injection selon la revendication 1, dans lequel le mécanisme de verrouillage est adapté pour empêcher le mouvement du porte-seringue vers l'ouverture de sortie lorsque le bouton de déclenchement est dans sa position en prise.

3. Dispositif d'injection selon la revendication 1 ou la revendication 2, dans lequel le bouton de déclenchement est situé au niveau d'une surface latérale externe du boîtier.

4. Dispositif d'injection selon la revendication 3, dans lequel la surface latérale externe est déplacée de l'axe longitudinal dans une direction perpendiculaire par rapport à l'axe longitudinal.

5. Dispositif d'injection selon l'une quelconque des revendications précédentes, dans lequel le bouton de déclenchement comprend une surface d'activation (102c) adaptée à l'application d'une pression par un utilisateur du dispositif d'injection dans une direction dans le boîtier pour déplacer le bouton de déclenchement de sa position en prise à sa position désolidarisée.

6. Dispositif d'injection selon la revendication 1, dans lequel la saillie comprend une ouverture (171) par laquelle s'étend le porte-seringue.

7. Dispositif d'injection selon la revendication 6, dans lequel l'ouverture comprend :
une première partie (171a) qui est dimensionnée pour venir en prise avec le porte-seringue lorsque le bouton de déclenchement est dans sa position en prise ; et
une seconde partie (171b) qui est dimensionnée pour ne pas venir en prise avec le porte-seringue lorsque le bouton de déclenchement est dans sa position désolidarisée.

8. Dispositif d'injection selon la revendication 7, dans lequel un bord (171c) de la première partie de l'ouverture vient en prise avec le porte-seringue lorsque le bouton de déclenchement est dans sa position en prise et n'est pas en prise lorsque le bouton de déclenchement est déplacé vers sa position désolidarisée.

9. Dispositif d'injection selon l'une quelconque des revendications 1 à 8, dans lequel le porte-seringue comprend une ouverture pour la mise en prise de la saillie lorsque le mécanisme de verrouillage est dans sa position en prise.

10. Dispositif d'injection selon la revendication 9, dans lequel l'ouverture est un canal s'étendant autour d'une section de la circonférence de la surface externe du porte-seringue.

11. Dispositif d'injection selon la revendication 9, dans lequel l'ouverture est une fente autour d'une section de la circonférence de la surface externe du porte-seringue et dans lequel la fente s'étend à travers la surface externe du porte-seringue.

12. Dispositif d'injection selon l'une quelconque des revendications 1 à 11, dans lequel une section du boîtier comprend une ouverture par laquelle s'étend la saillie, au moins lorsque le mécanisme de verrouillage est dans sa position en prise.

13. Dispositif d'injection selon l'une quelconque des revendications précédentes, dans lequel l'entraînement comprend un arbre s'étendant parallèlement à l'axe longitudinal.

14. Dispositif d'injection selon l'une quelconque des revendications précédentes, dans lequel l'actionneur comprend des moyens de sollicitation adaptés pour faire passer le porte-seringue d'une position rétractée à une position étendue.

15. Dispositif d'injection selon l'une quelconque des revendications précédentes, comprenant en outre un capuchon amovible (111) destiné à être placé sur le boîtier et à couvrir au moins une partie de l'ouverture de sortie.

16. Dispositif d'injection selon la revendication 15, dans lequel le capuchon amovible est relié à un dispositif de protection amovible (123) sur la buse de décharge, de telle sorte que le dispositif de protection est retiré de la buse de décharge lorsque le capuchon est retiré du boîtier.

17. Dispositif d'injection selon l'une quelconque des revendications précédentes, dans lequel l'entraînement comporte des premier et second éléments d'entraînement (132, 134), dont le premier est actionné par l'actionneur et agit à son tour sur le second, et le second agit sur la seringue ou le porte-seringue pour le faire avancer de sa position rétractée à sa position étendue et décharger son contenu par la buse de décharge, le premier élément d'entraînement étant capable de se déplacer par rapport au second lorsque le premier est actionné par l'actionneur et que le second est retenu par la seringue ou le porte-seringue.

18. Dispositif d'injection selon la revendication 17, comprenant en outre un accouplement qui empêche le premier élément d'entraînement de se déplacer par rapport au second jusqu'à ce qu'ils aient été avancés à une position nominale de désaccouplement qui est moins avancée que ladite position nominale de relâchement.

19. Dispositif d'injection selon la revendication 17, dans lequel l'accouplement comprend un mécanisme de désaccouplement, activé lorsque les éléments d'entraînement ont été avancés jusqu'à ladite position nominale de désaccouplement et adapté pour désaccoupler le premier élément d'entraînement du second, permettant ainsi au premier élément d'entraînement de se déplacer par rapport au second.
